# EUROPEAN PATENT SPECIFICATION

(11) **EP 4 096 530 B1**
(45) Date of publication and mention of the grant of the patent: **22.10.2025**
(21) Application number: 21747591.2
(22) Date of filing: 29.01.2021
(51) Int. Cl.: A61B 17/04, A61B 17/06, A61F 2/08

(54) **KNOTLESS SOFT TISSUE IMPLANT SYSTEMS**
KNOTENLOSE WEICHTEILIMPLANTATSYSTEME
SYSTÈMES D'IMPLANT DE TISSUS MOUS SANS NOEUD

(30) Priority: 31.01.2020 US 202062968765 P
(43) Date of publication of application: 07.12.2022
(73) Proprietor: Paragon 28, Inc., Englewood, CO 80112 (US)
(72) Inventor: HARTSON, Kyle James, Englewood, Colorado 80112 (US)
(74) Representative: Murgitroyd & Company
(86) International application number: PCT/US2021/015709
(87) International publication number: WO 2021/155148

(56) References cited:
- EP-A1- 2 698 117
- EP-A2- 2 596 755
- WO-A1-2018/085663
- US-A1- 2006 259 076
- US-A1- 2012 150 297
- US-A1- 2014 163 614
- US-A1- 2015 173 739
- US-A1- 2016 051 246
- US-A1- 2016 220 243
- US-A1- 2017 095 363
- US-A1- 2018 125 638
- US-A1- 2018 353 167

## Description

### CROSS REFERENCE TO RELATED APPLICATION

This application claims benefit of priority of U.S. Provisional Patent Application No. 62/968,765, filed January 31, 2020, and entitled "Knotless Soft Tissue Implant Systems and Related Methods,".

### TECHNICAL FIELD

The present disclosure relates generally to implants and methods for repair of soft tissue and/or bone. More specifically, but not exclusively, the present disclosure relates to knotless implants, systems, assemblies and related methods for joining soft tissue to soft tissue, soft tissue to bone, and bone to bone.

### BACKGROUND

Tissues and/or bones of a mammalian body (e.g., a human body) may become torn or otherwise be segmented or separated such that joining of the portions of the tissues and/or bones may be anatomically and/or physiologically advantageous or desirable.

For example, the human foot includes a plantar plate on the bottom of the foot under a metatarsophalangeal joint (MTP joint). The plantar plate is a thick ligamentous (fibrocartilaginous) structure that attaches a metatarsal bone and a corresponding proximal phalanx bone. A plantar plate may become torn or otherwise compromised. In some instances, the tissue of the plantar plate itself may become attenuated, tear or otherwise become segmented along its length between corresponding metatarsal and proximal phalanx bones. Alternatively, a plantar plate may tear from or otherwise become decoupled from the corresponding metatarsal or proximal phalanx bone(s).

As another example, the human hand includes a palmar plate. The palmar plate is an analogous structure to the plantar plate. A palmar plate is associated with each metacarpophalangeal joint (MCP joint) and each interphalangeal joint in the hand. Like the plantar plate, the palmar plate may tear.

Many other ligaments of a mammalian body may become torn, such as the tissue itself or the attachment of the ligament to one or more bones. Similarly, one or more bones of a mammalian body may become broken or otherwise segmented. Typical tissue and bone repair options include suturing/re-approximating the tear or break, or re-attaching the tissue and bone, to induce healing. Current systems for such repairs tend to be bulky, complex and/or require multiple and/or relatively large through holes to be formed in the tissue and/or bone.

US 2015/173739 A1 discloses a knotless tissue repair assembly for attachment of tissue to bone including an anchoring implant with a length of suture threaded therethrough. The implant is preferably a soft flexible three-dimensional structure. The implant may be actuated from a first elongate low profile shape into a second short radially expanded shape having a larger diameter than the hole through which it was placed. The suture extends through the anchor, through a tissue to be secured, and back through a designated suture-binding region or passageway within the anchor enabling the suture to be secured therein and without the need for a physician to tie a knot. Further tension applied to a suture leg approximates the tissue to the anchor until a desired tension or distance between the tissue and anchor is achieved.

WO 2018/085663 A1, EP 2596755 A2, US 2012/150297 A1 and EP 2698117 A1 disclose further systems of the prior art.

Thus, there is a need for implants, systems, assemblies and methods for plantar plate (and palmar plate) repair, and joining of other tissues and/or bones of the foot, hand and other parts of a mammalian (e.g., human) body, that are compact, maneuverable and simple to use. There is also a need for tissue and/or bone repair/joining implants, systems, assemblies and methods that do not require a plurality of and/or relatively large through holes formed in the joined tissue and/or bone segments.

### SUMMARY

The invention is defined by appended claim 1. The present disclosure is directed toward devices and methods for joining tissue and/or bone segments or portions, such as soft tissue to soft tissue, soft tissue to bone, and bone to bone. The implants, systems, assemblies and methods for joining soft tissue to soft tissue, soft tissue to bone, and bone to bone may be used for repairing a torn plantar plate (and/or palmar plate). However, the implants, systems, assemblies and methods may be equally employed to repair/join any other tissue and/or bone segments or portions of the foot, of the hand or of other parts of a mammalian body (e.g., a human body).

In one aspect, the present disclosure provides a bone and/or tissue joining implant system, comprising a flexible anchor tube, a suture and at least one suture passer. The flexible anchor tube comprises an annular side wall defining an internal cavity, a first longitudinal end and a second longitudinal end. The suture extends through the side wall of the anchor tube a plurality of times and forms a free loop portion that extends from the side wall. The at least one suture passer extends through a portion of the loop portion of the suture and is configured to translate first and second end portions of the suture through the loop portion via translation of the at least one suture passer. The suture extends along a pattern that allows the suture to slide through the anchor tube when the first and second end portions of the suture are tensioned.

In some embodiments, the first end portion of the suture extends from a first end of the suture and through a first portion of the side wall of the anchor tube from an exterior surface of the anchor tube proximate to the first longitudinal end thereof into the first internal cavity, extends longitudinally through a portion of the internal cavity toward the second longitudinal end of the anchor tube, and extends through a second portion of the side wall distal to the first longitudinal end from the internal cavity to the exterior surface of the anchor tube. In some such embodiments, the second end portion of the suture extends from a second end of the suture and through a third portion of the side wall from an exterior surface of the anchor tube proximate to the second longitudinal end thereof into the internal cavity, extends longitudinally through a portion of the internal cavity toward the first longitudinal end, and extends through a fourth portion of the side wall distal to the second longitudinal end from the internal cavity to the exterior surface of the anchor tube. In some such embodiments, a first intermediate portion of the suture extends from the first end portion that extends through the second portion of the side wall of the anchor tube, and a second intermediate portion of the suture extends from the second end portion that extends through the fourth portion of the side wall of the anchor tube, and wherein the first and second intermediate portions of the suture form the loop portion. In some such embodiments, the first and second intermediate portions of the suture are contiguous portions of the suture.

In accordance with the invention, tensioning of the first and second end portions of the suture deforms the anchor tube from a neutral configuration into a deformed configuration. In some such embodiments, in the deformed configuration, the anchor tube defines a curved shape. In some such embodiments, the concave shape comprise a U-shape or a V-shape. In accordance with the invention, the anchor tube is folded over upon itself along a longitudinal length thereof in the deformed configuration. In some other such embodiments, the anchor tube defines a first maximum lateral width and a first maximum longitudinal length in the neutral configuration, and defines a second maximum lateral width that is greater than the first maximum lateral width and a second maximum longitudinal length that is less than the first maximum longitudinal length in the deformed configuration.

In some embodiments, the anchor tube is longitudinally elongated in the neutral configuration. In some such embodiments, the anchor tube is cylindrical in the neutral configuration.

In some embodiments, the anchor tube is formed from a biocompatible material. In some embodiments, the suture is formed from a biocompatible material.

In some embodiments, the at least one suture comprises a multifilament suture. In some such embodiments, the suture is a braided suture.

In some embodiments, the suture is non-hollow. In some embodiments, the system further comprises at least one insertion device. In some such embodiments, the first and second end portions of the suture are coupled to the at least one insertion device. In some such embodiments, the at least one insertion device comprises a first insertion device coupled to the first end portion of the suture, and a second insertion device coupled to the second end portion of the suture.

In some embodiments, the at least one insertion device comprises at least one needle. In some such embodiments, the at least one needle comprises at least one non-linear needle.

In some embodiments, the anchor member is flexible. In some embodiments, the anchor member is formed of a hollow braided suture. In some embodiments, the at least one suture passer comprises a first suture passer and a second suture passer.

In some such embodiments, the first suture passer extends through a first portion of the loop portion of the suture, and the second suture passer extends through a second portion of the loop portion of the suture. In some such embodiments, the first suture passer and the second suture passer extend through a common portion of the loop portion of the suture. In some embodiments, the first suture passer passes through the loop portion of the suture along a first direction, and the second suture passer passes through the loop portion of the suture along a second direction that substantially opposes the first direction.

In some embodiments, the at least one suture passer comprises an exposed loop. In some embodiments, when the first and second end portions of the suture are translated through the loop portion along a first direction via the suture passer and the first and second end portions are tensioned, the loop portion applies tension on the first and second end portions sufficient to prevent the first and second end portions from translating through the loop portion along a second direction that opposes the first direction.

In another aspect, not forming part of the claimed invention, a method of joining first and second anatomical structures is provided. The method comprises implanting a flexible anchor tube comprising an annular side wall defining an internal cavity, a first longitudinal end and a second longitudinal end in the first anatomical structure. The method also comprises implanting a portion of the first and second end portions of a suture through a portion of the second anatomical structure, the suture forming a free loop portion that extends from the side wall, and wherein the suture extends along a pattern that allows the suture to slide through the anchor tube when the first and second end portions of the suture are tensioned. The method further comprises passing a portion of the first and second end portions of the suture through at least one loop of at least one suture passer that extend through a securement portion of the loop portion. The method also comprises passing a portion of the first and second end portions of the suture through the securement portion of the loop portion via the at least one suture passer. The method further comprises tensioning the first and second end portions of the suture to shorten a length of the loop portion, deform the anchor tube into a deformed configuration, and approximate the first and second anatomical structures. The securement portion of the loop portion secures the position of the first and second end portions within the securement portion subsequent to the tensioning.

These and other objects, features and advantages of the inventions provided by this disclosure will become apparent from the following detailed description of the various aspects of the invention taken in conjunction with the accompanying drawings.

### BRIEF DESCRIPTION OF THE DRAWINGS

The accompanying drawings, which are incorporated in and constitute a part of the specification, illustrate embodiments of the inventions and together with the detailed description herein, serve to explain the principles of the inventions. It is emphasized that, in accordance with the standard practice in the industry, various features may or may not be drawn to scale. In fact, the dimensions of the various features may be arbitrarily increased or reduced for clarity of discussion. The drawings are only for purposes of illustrating embodiments of inventions of the disclosure and are not to be construed as limiting the inventions.
**FIG. 1** is a top view of a tissue and/or bone joining implant system, in accordance with the present disclosure;
**FIG. 2** is a side cross-sectional view of an anchor tube and an associated suture portion of the implant system of FIG. 1, in accordance with the present disclosure;
**FIG. 3** is a top perspective view of the implant system of FIG. 1, in accordance with the present disclosure;
**FIG. 4** is a bottom perspective view of the implant system of FIG. 1, in accordance with the present disclosure;
**FIG. 5A** is a side cross-sectional view illustrating the anchor tube of the implant system of FIG. 1 in a deformed state and pulled against an inner surface of a bone and extending through a tissue, in accordance with an aspect of the present invention;
**FIG. 5B** is a side cross-sectional view illustrating the anchor tube of the implant system of FIG. 1 in a deformed state and securing the bone and tissue of FIG. 5A together with the overlapping suture portions seated on the exterior of the bone, in accordance with an aspect of the present invention;
**FIG. 5C** is a side cross-sectional view illustrating the anchor tube of the implant system of FIG. 1 in a deformed state and securing the bone and tissue of FIG. 5A together with the overlapping suture portions seated against the anchor tube, in accordance with an aspect of the present invention;
**FIG.** 6 is a top view illustrating a pair of the implant systems of FIG. 1 repairing a tissue, in accordance with an aspect of the present invention;
**FIG.** 7 is a top view illustrating the implant system of FIG. 6 implanted through a bone, in accordance with an aspect of the present invention;
**FIG.** 8 is a side cross-sectional view illustrating the implant system of FIG. 7 with the anchor tube loaded on an inserter, in accordance with an aspect of the present invention;
**FIG. 9** is a side cross-sectional view illustrating the implant system of FIG. 8 with the anchor tube implanted in a bone, in accordance with an aspect of the present invention;
**FIG. 10** is a side cross-sectional view illustrating the implant system of FIG. 9 with the anchor tube implanted in a bone and the end portions of the suture implanted through a tissue, in accordance with an aspect of the present invention;
**FIG. 11** is a side cross-sectional view illustrating the implant system of FIG. 10 with the anchor implanted in a bone, the end portions of the suture implanted through the tissue, and the end portion of the suture passed through the suture loop portion, in accordance with an aspect of the present invention; and
**FIG. 12** is a side cross-sectional view illustrating the implant system of FIG. 11 securing the bone and tissue together.

### DETAILED DESCRIPTION

In this detailed description and the following claims, the words proximal, distal, anterior or plantar, posterior or dorsal, medial, lateral, superior and inferior are defined by their standard usage for indicating a particular part or portion of a bone or implant according to the relative disposition of the natural bone or directional terms of reference. For example, "proximal" means the portion of a device or implant nearest the torso, while "distal" indicates the portion of the device or implant farthest from the torso. As for directional terms, "anterior" is a direction towards the front side of the body, "posterior" means a direction towards the back side of the body, "medial" means towards the midline of the body, "lateral" is a direction towards the sides or away from the midline of the body, "superior" means a direction above and "inferior" means a direction below another object or structure. Further, specifically in regards to the foot, the term "dorsal" refers to the top of the foot and the term "plantar" refers the bottom of the foot.

Similarly, positions or directions may be used herein with reference to anatomical structures or surfaces. For example, as the current implants, devices, instrumentation and methods are described herein with reference to use with the bones of the foot, the bones of the foot, ankle and lower leg may be used to describe the surfaces, positions, directions or orientations of the implants, devices, instrumentation and methods. Further, the implants, devices, instrumentation and methods, and the aspects, components, features and the like thereof, disclosed herein are described with respect to one side of the body for brevity purposes. However, as the human body is relatively symmetrical or mirrored about a line of symmetry (midline), it is hereby expressly contemplated that the implants, devices, instrumentation and methods, and the aspects, components, features and the like thereof, described and/or illustrated herein may be changed, varied, modified, reconfigured or otherwise altered for use or association with another side of the body for a same or similar purpose without departing from the scope of the invention. For example, the implants, devices, instrumentation and methods, and the aspects, components, features and the like thereof, described herein with respect to the right foot may be mirrored so that they likewise function with the left foot. Further, the implants, devices, instrumentation and methods, and the aspects, components, features and the like thereof, disclosed herein are described with respect to the foot for brevity purposes, but it should be understood that the implants, devices, instrumentation and methods may be used with other bones of the body having similar structures.

Generally stated, disclosed herein are implants, systems, assemblies and methods for joining soft tissue to soft tissue, soft tissue to bone, and bone to bone. The implants, systems, assemblies and methods may be used for repairing a torn plantar plate of a metatarsophalangeal joint (MTP joint). While the implants, systems, assemblies and methods may be illustrated and described in the present disclosure in the context of plantar plate repair, the implants, systems, assemblies and methods may equally be employed or may be adapted without undue experimentation to join any soft tissue to any soft tissue, any soft tissue to any bone, or any bone to any bone. For example, the implants, systems, assemblies and methods may be equally employed to repair/join any other tissue and/or bone segments or portions of the foot or other parts of the mammalian (e.g., human) body, such as but not limited to a torn palmar plate.

The implants, systems, assemblies and related methods for joining soft tissue to soft tissue, soft tissue to bone, and bone to bone of the present disclosure may be similar to, such as include at least one feature or aspect of, the implants, systems, assemblies and related methods disclosed in U.S. Provisional Patent Application No. 62/775,591, filed on December 5, 2018, and entitled Implant System and Methods of Use, U.S. Provisional Patent Application No. 62/883,429, filed on August 6, 2019, and entitled Soft Tissue Implant Systems, Instruments and Related Method and/or International PCT Application No. PCT/US2019/064741, filed on December 5, 2019, and entitled Soft Tissue Implant Systems, Instruments and Related Methods. Similarly, the implants, systems, assemblies and related methods for joining soft tissue to soft tissue, soft tissue to bone, and bone to bone of the present disclosure may include one or more instrument (e.g., one or more insertion and/or implantation instrument) disclosed in U.S. Provisional Patent Application No. 62/883,429, filed on August 6, 2019, and entitled Soft Tissue Implant Systems, Instruments and Related Method and/or International PCT Application No. PCT/US2019/064741, filed on December 5, 2019, and entitled Soft Tissue Implant Systems, Instruments and Related Methods.

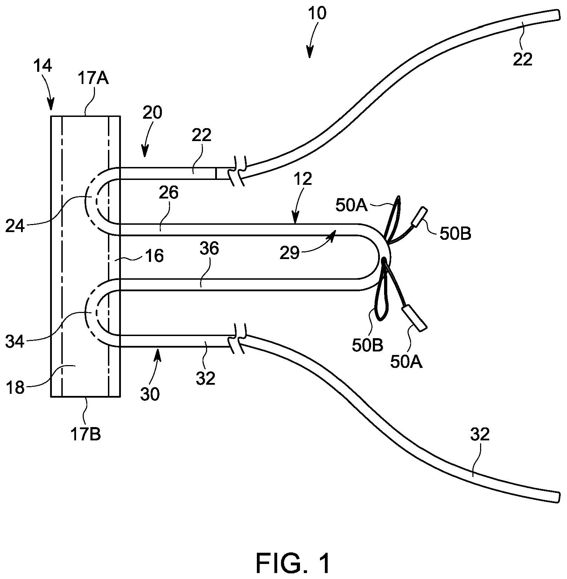

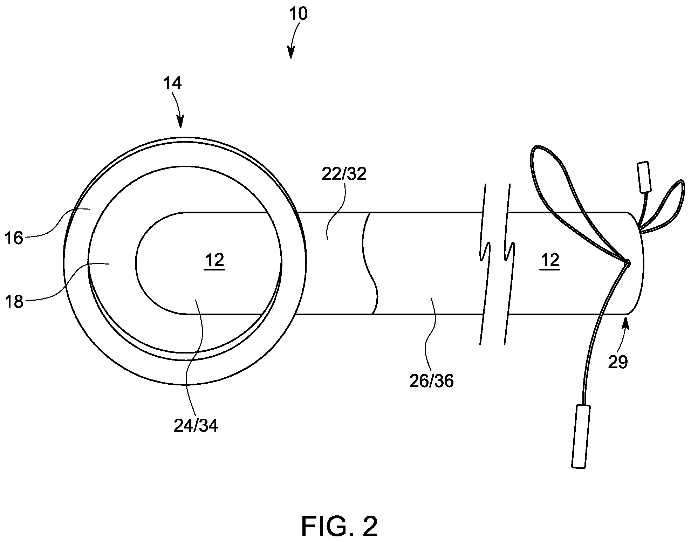

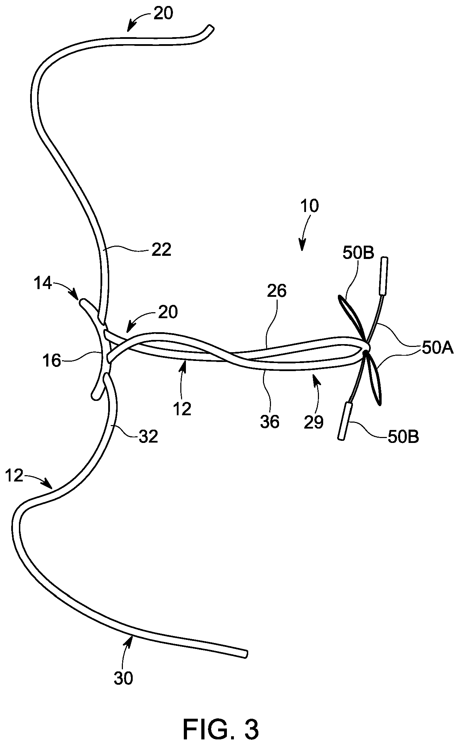

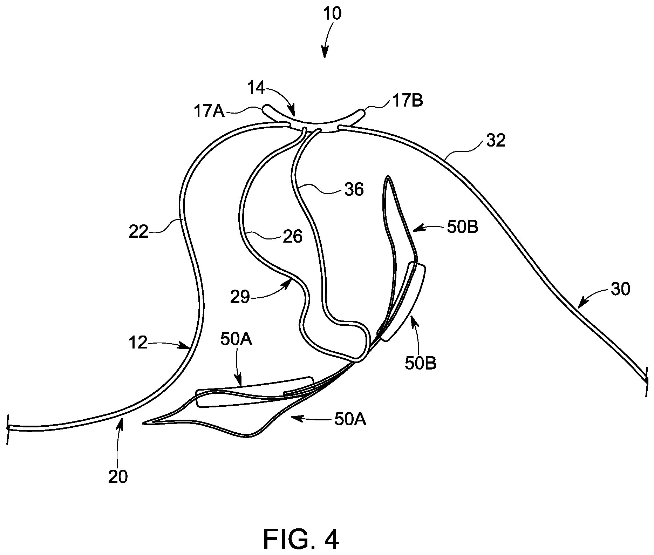

Referring to the drawings, wherein like reference numerals are used to indicate like or analogous components throughout the several views, and with particular reference to FIGS. 1-12, there is illustrated an exemplary embodiment of an implant system 10 for joining soft tissue to soft tissue, soft tissue to bone, or bone to bone according to the present disclosure. As shown in FIGS. 1-5C, the implant system 10 may include a first biocompatible collapsible anchor tube 14 (also referred to herein as a "tensioning anchor 14"), and a biocompatible suture 12 (also referred to herein as a "tensioning strand 12") that passes through a side wall 16 of the anchor tube 14 a plurality of times.

As shown in FIGS. 1-5C, the first biocompatible collapsible anchor tube 14 may be elongated along a longitudinal direction. In some embodiments, the anchor tube 14 may define a longitudinal length within the range of 5 mm and 35 mm, or within the range of 8 mm and 17 mm, or within the range of 10 mm and 15 mm. In the illustrative exemplary embodiment, the anchor tube 14 defines a longitudinal length of about ½ inch (1.27 cm). The anchor tube 14 may be sized proportionally bigger than suture 12. For example, the width or diameter of the anchor tube 14 may be larger than that of the suture 12.

The anchor tube 14 may be formed of an annular side wall 16 that defines an inner cavity 18 and first and second longitudinal ends 17A, 17B, as shown in FIG 1. The anchor tube 14 may thereby be hollow. The size of the inner cavity 18 may be larger than the suture 12 such that the suture 12 is able to pass though the inner cavity 18. The inner cavity 18 may extend through the anchor tube 14 such that the inner cavity 18 is open or accessible at one or both of the longitudinal ends 17A, 17B of the anchor tube 14. In some embodiments, as shown in FIG. 1, the inner cavity 18 may extend entirely through the anchor tube 14 such that the inner cavity 18 is open or accessible at both of the longitudinal ends 17A, 17B of the anchor tube 14. In some alternative embodiments (not shown), the inner cavity 18 may extend only partially through the anchor tube 14 such that the inner cavity 18 is open or accessible at only one of the longitudinal ends 17A, 17B of the anchor tube 14. In such embodiments, one of the longitudinal ends 17A, 17B of the anchor tube 14 (and the inner cavity 18) may thereby be closed via an end wall, plug, heat seal or any other configuration (while another longitudinal end of the anchor tube 14 may be open to the inner cavity 18). In some other alternative embodiments (not shown), both of the longitudinal ends 17A, 17B of the anchor tube 14 (and the inner cavity 18) may be closed. In this way, the inner cavity 18 may be sealed or closed off within the anchor tube 14.

The anchor tube 14 may be flexible, collapsible, deformable, bendable, stretchable or otherwise re-arrangeable in overall shape. For example, in a natural, neutral or non-deformed state as shown in FIGS. 1-4, the anchor tube 14 may extend substantially linearly or straight along the longitudinal direction. The anchor tube 14 may thereby be substantially cylindrical in its natural state, as shown in FIGS. 1-4. In some embodiments, in the natural state as shown in FIGS. 1-4, the anchor tube 14 may extend substantially linearly or straight along the longitudinal direction, or include a slightly curved or arcuate shape. The anchor tube 14 may thereby be substantially cylindrical or slightly curved in its natural state, as shown in FIGS. 1-4. In the natural state, the anchor tube 14 defines a first maximum lateral width and a first maximum longitudinal length. As explained further below, the anchor tube 14 may be deformed via tensioning of the suture 12 into a deformed shape, configuration or state in which the anchor tube 14 defines a second maximum lateral width that is greater than the first maximum lateral width and a second maximum longitudinal length that is less than the first maximum longitudinal length.

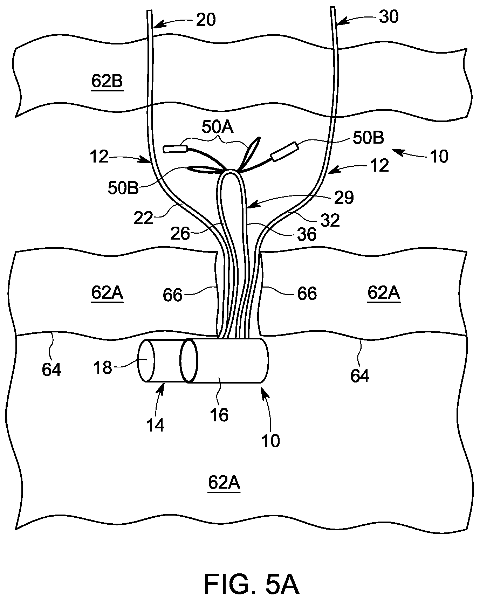

From the natural state, at least the side wall 16 of the anchor tube 14 may be deformed inwardly such that the inner cavity 18 is at least partially collapsed along the lateral direction. The anchor tube 14 (e.g., at least the side wall 16 thereof) may also be deformed such that the anchor tube 14 is curved, bent, compressed or otherwise rearranged from the substantially linear or cylindrical shape in the neutral state to a "bunched" shape. As shown in FIGS. 5A-7, the suture 12 may extend through a side or portion of the side wall 16 a plurality of times which may form the anchor tube 14 into a concave/convex (depending with respect to the portion of the side wall 16 including the suture 12 extending therethrough), arcuate, folded, "C" or "U" and/or "V" shape. For example, the suture 12 may collapse, compress or pull together the side or portion of the side wall 16 such that the anchor tube 14 assumes a tightly arcuate (e.g., defined by a relatively small radius) or U-shape, as shown in FIGS. 5A-7.

The anchor tube 14 (e.g., the side wall 16) may be solid or porous. For example, the anchor tube 14 (e.g., the side wall 16) may be molded via a solid material. As another example, the anchor tube 14 (e.g., the side wall 16) may be woven from one or more threads, fibers or filaments. For example, in some embodiments, the anchor tube 14 is formed of braided or woven filaments, yarn or sutures. In one such embodiment, the anchor tube 14 is formed of a hollow braided suture. In some embodiments, the anchor tube 14 (e.g., the side wall 16) may be formed via one or more machining, additive and/or extrusion formation process. The anchor tube 14 (e.g., the side wall 16) may be of one-piece construction or formed of a plurality of members or portions (and/or materials). The anchor tube 14 (e.g., the side wall 16) may be formed or made from any biocompatible material(s). For example, the anchor tube 14 (e.g., the side wall 16) may be made or formed of polyester, polyethylene (e.g., ultra-high-molecular-weight polyethylene), polypropylene, nylon (e.g., poylamide), silk, polyglycolic acid (PGA), polydioxanone (PDO), polylactic acid (PLA), polylactic-co-glycoic acid (PLGA), stainless steel, or a combination thereof. In one example, the anchor tube 14 is formed of polyester.

As noted above, the biocompatible suture 12 passes through a side or portion of the side wall 16 of the anchor tube 14 a plurality of times. The suture 12 may be any flexible biocompatible thread, fiber, filament or like member. The suture 12 may be a monofilament suture or a multifilament suture (e.g., a multifilament braided structure). For example, the suture 12 may be a cobraid of two filaments formed of the same or differing materials. The suture 12 may be made or formed of any biocompatible material, such as any non-absorbable biocompatible material. In some such embodiments, the suture 12 may be made or formed of polypropylene, polyethylene (e.g., ultra-high-molecular-weight polyethylene), nylon (e.g., poylamide), polyester, polyvinylidene difluoride (PVDF), polyglycolic acid (PGA), polydioxanone (PDO), polylactic acid (PLA), polylactic-co-glycoic acid (PLGA), silk, stainless steel or a combination thereof. For example, the suture 12 may be a cobraid of a polyethylene (e.g., ultra-high-molecular-weight polyethylene) filament and a polyester filament. In one example, the suture 12 is formed of polyethylene.

The suture 12 may be non-hollow, solid, continuous or otherwise void of an internal longitudinally extending cavity or open space. For example, in cross-section, the suture 12 may be comprised of a plurality of filaments that are intertwined, braded or woven throughout the cross-section. The suture 12 may thereby be non-hollow.

The suture 12 may be any cross-sectional shape. The suture 12 may be any size, such as any length and/or cross-sectional width or diameter. In some embodiments, the suture 12 may be at least 45 cm long, such as within range of 10 cm to 100 cm, or more preferably within range of 40 cm to 90 cm. The suture 12 may be at least a size 6-0 United States Pharmacopeia (USP) designated non-absorbable suture, such as a size 6-0 to 5 USP designated non-absorbable suture, or more preferably, a 4-0 to 1 USP designated non-absorbable suture. In some embodiments, the suture 12 may be at least 60 cm long and at least a size 0 USP designated non-absorbable suture, or at least 45 cm long and at least a size 2-0 USP designated non-absorbable suture.

As discussed above, the suture 12 of the implant system 10 is configured or arranged in a pattern that allows the suture 12 to slide freely through the anchor tube 14. The implant system 10 is configured such that tension applied to the first and second ends or end portions 20, 30 of the suture 12 extending from the anchor tube 14 causes the suture 12 to slide through the anchor tube 14 as the length of the suture 12 extending through the anchor tube 14 is shortened, and the anatomical structures coupled with the anchor tube 14 and the first and second end portions 20, 30 of the suture 12 are drawn together.

As shown in FIG. 1, the first and second end portions 20, 30 of the suture 12 may each extend through the side wall 16 of the anchor tube 14. The first end portion 20 of the suture 12 may include a first end suture segment 22 that extends from the free end of the suture 12 and through the side wall 16 of the anchor tube 14 into the interior cavity 18. As shown in FIG. 1, the first end suture segment 22 of the first end portion 20 of the suture 12 may extend through a first portion or side of the side wall 16 proximate to the first longitudinal end 17A of the anchor tube 14. Inside of the interior cavity 18, a first interior portion 24 of the first end portion 20 of the suture 12 may extend from the first end suture segment 22 and longitudinally within the interior cavity 18 toward the second longitudinal end 17B of the anchor tube 14 (that opposes the first longitudinal end 17A), as shown in FIG. 1. The first interior portion 24 of the first end portion 20 may extend through the first portion or side of the side wall 16 distal to the first longitudinal end 17A (and proximate to the second longitudinal end 17B) of the anchor tube 14. In an alternative embodiment (not shown), the first interior portion 24 of the first end portion 20 of the suture 12 may extend from the first end suture segment 22 and longitudinally within the interior cavity 18 toward the first longitudinal end 17A of the anchor tube 14 (that opposes the second longitudinal end 17B). The first interior portion 24 of the first end portion 20 may thereby extend through the first portion or side of the side wall 16 proximate to the second longitudinal end 17B (and proximate to the first longitudinal end 17A) of the anchor tube 14.

As shown in FIGS. 1-5C, a medial portion of the suture 12 may extend from and between two portions of the side wall 16 and form a loop portion 29. The anchor tube 14 may be slidably adjustable along the suture 12 via tension applied to the first and second end portions 20, 30 such that the anchor tube 14 is slid along the length of the suture 12, and the loop portion 29 shortened. The length of the loop portion of the suture 12 and the length of the first and second end portions 20, 30 extending from the side wall 16 of the anchor tube 14 can thereby be adjusted. In this way, the first anchor tube 14 and the first and second end portions 20, 30 can be implanted into bone and/or tissue segments or portions, and the first and second end portions 20, 30 tensioned to reduce the loop portion 29, deform the anchor tube 14, and join/repair the segments or portions and draw them together. As explained further below, such a configuration of the suture 12 and anchor tube 14, and thereby the bone and/or tissue segments or portions, may be maintained or secured by passing the first and second end portions 20, 30 of the suture 12 through a portion of the loop portion 29 of the suture 12. As the system/construct 10 tightens due to the tensioning of the first and second end portions 20, 30, the loop portion 29 may also tighten and thereby prevent the first and second end portions 20, 30 from sliding therethrough/therefrom. In this way, the system/construct 10 may be self-locking/securing, and not require any knots or external mechanisms to lock or secure the system/construct 10 and the configuration of the bone and/or tissue segments or portions.

As shown in FIGS. 1-4, the second end portion 30 of the suture 12 may include a second end suture segment 32 that extends from the free end of the suture 12 and through the side wall 16 of the anchor tube 14 into the interior cavity 18. As shown in FIG. 1, the second end suture segment 32 of the second end portion 30 of the suture 12 may extend through a first portion or side of the side wall 16 proximate to the second longitudinal end 17B of the anchor tube 14. Inside of the interior cavity 18 of the anchor tube 14, a second interior portion 34 of the second end portion 30 of the suture 12 may extend from the second end suture segment 32 and longitudinally within the interior cavity 18 toward the first longitudinal end 17A of the anchor tube 14 (that opposes the second longitudinal end 17B), as shown in FIG. 1. The second interior portion 34 of the second end portion 30 may extend through the first portion or side of the side wall 16 distal to the first longitudinal end 17A (and proximate to the second longitudinal end 17B) of the anchor tube 14. In an alternative embodiment (not shown), the second interior portion 34 of the second end portion 30 of the suture 12 may extend from the second end suture segment 32 and longitudinally within the interior cavity 18 toward the second longitudinal end 17B of the anchor tube 14 (that opposes the first longitudinal end 17A. The second interior portion 34 of the second end portion 30 may thereby extend through the first portion or side of the side wall 16 distal to the second longitudinal end 17B (and proximate to the first longitudinal end 17A) of the anchor tube 14.

As shown in FIGS. 1-4, a first intermediate portion 26 of the suture 12 extends from the first interior portion 24 of the suture 12 and the side wall 16 of the anchor tube 14, and a second intermediate portion 36 of the suture 12 extends from the second interior portion 34 of the suture 12 and the side wall 16 of the anchor tube 14. The first intermediate portion 26 and the second intermediate portion 36 cooperate to form the exposed loop portion 29 positioned exterior to the anchor tube 14 (i.e., exterior to the interior cavity 18). The loop portion 29 may thereby comprise a medial portion of the suture 12 that extends from a first portion of the exterior surface of the side wall 16 of the anchor tube 14 and back through a second portion of the exterior surface of the side wall 16 of the anchor tube 14 that is spaced along the longitudinal length of the anchor tune 14.

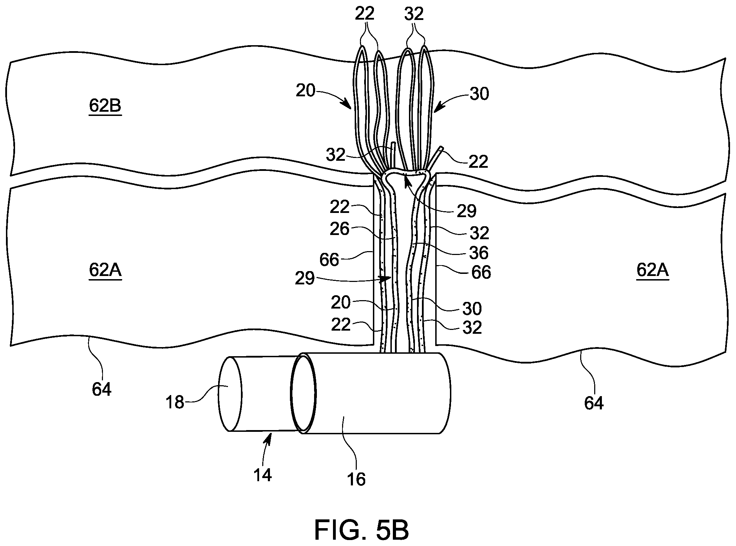

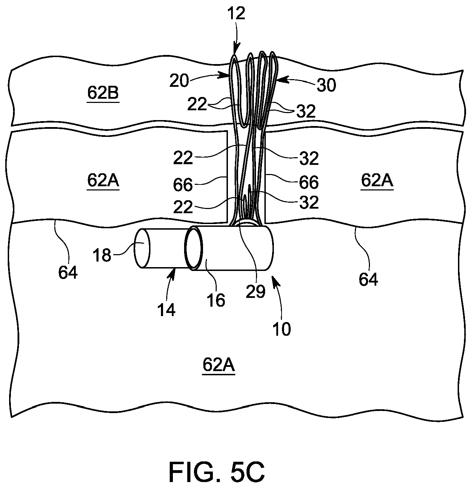

As shown in FIGS. 1-5A, the system/construct 10 also comprises at least one suture passer 50A, 50B extending through a portion of the loop portion 29 of the suture 12 configured to translate the first and second end segments 22, 32 of the first and second end portions 20, 32, respectively, of the suture 12 through the loop portion 29 via translation of the at least one suture passer 50A, 50B through the loop portion 29. In some embodiments, the at least one suture passer 50A, 50B may include an exposed tail portion and an exposed loop or hook through which the first and second end segments 22, 32 of the first and second end portions 20, 32 may be passed through, as shown in FIGS. 1-5A. The at least one suture passer 50A, 50B may pass through the loop portion 29 such that the tail and loop portions of the at least one suture passer 50A, 50B extend from differing sides or portions of the outer/exterior surface of the suture 12 of the loop portion 29, as shown in FIGS. 1-5A. the first and second end segments 22, 32 of the first and second end portions 20, 32 may thereby be passed through the loop portion of the at least one suture passer 50A, 50B, and the tail portion of the at least one suture passer 50A, 50B may be pulled-tensioned to translate/pull the first and second end segments 22, 32 through the respective portion of the loop portion 29 (i.e., through a portion of the suture 12 of the loop portion 29), as shown in FIGS. 5B and 5C.

The suture 12 is configured such that when the first and second end segments 22, 32 of the first and second end portions 20, 32 are translated/pulled through the portion(s) of the loop portion 29 and the suture is tensioned (via tensioning of the first and second end portions 20, 32), the loop portion 29 (is shortened and creates sufficient friction and/or compression on the first and second end segments 22, 32 that the first and second end segments 22, 32 are prevented from naturally translating or sliding back through the loop portion 29, as shown in FIGS. 5B and 5C. The system/construct 10 may thereby be selfsecuring or locking in that the first and second end portions 20, 32 of the suture 12 do not need to be knotted or secure via an external fixation mechanism to secure the configuration of the system/construct 10, and thereby the anatomical structures (e.g., bone(s) and/or tissue(s)) coupled by the anchor tube 14 and the first and second end segments 22, 32 of the suture 12, as explained further below and as shown in FIGS. 5B and 5C.

As shown in FIGS. 1-5A, the illustrated exemplary embodiment of the system/construct 10 includes a first suture passer 50A and a second suture passer 50B. In some embodiments, the first suture passer 50A extends through a first portion of the loop portion 29 of the suture 12, and the second suture passer 50B extends through a second portion of the loop portion 29 of the suture 12 that differs from the first portion. In some other embodiments, as shown in FIGS. 1-5C, the first suture passer 50A and the second suture passer 50B extend through a common portion of the loop portion 29 of the suture 12. In some embodiments, the first suture passer 50A and/or the second suture passer 50B extend directly laterally through the suture 12 of the loop portion 29. In some other embodiments, the first suture passer 50A and/or the second suture passer 50B may extend through a longitudinal portion of the suture 12 of the loop portion 29. In some embodiments, the suture 12 may comprise a braid of filaments (as discussed above), and the first suture passer 50A and the second suture passer 50B may extend through a portion of the braded filaments.

As shown in FIGS. 1-5A, the first suture passer 50A may extend through the loop portion 29 of the suture 12 along a first direction such that a loop or hook portion thereof is positioned on a first side of the loop portion 29, and the second suture passer 50B may extend through the loop portion 29 along a second direction that differs from the first direction (e.g., substantially opposes the first direction) such that a loop or hook portion thereof is positioned on a second side of the loop portion 29 that differs from the first side thereof.

To facilitate implantation of the implant, the first and second end segments 22, 32 of the suture 12 may be coupled or attached (e.g., tied, swaged or otherwise removably or fixedly coupled) to a needle (e.g., a curved or twisted needle) or other insertion device (not shown). The insertion device may thereby be utilized to pass or translate at least a portion of the first and second end segments 22, 32 of the first and second end portions 20, 32 through an anatomical structure. In some embodiments, the first end segment 22 of the first portion 20 of the suture 12 is coupled to a first insertion device (e.g., a first needle), and the second end segment 32 of the second portion 30 of the suture 12 is coupled to a second insertion device (e.g., a second needle).

In some embodiments (not shown), the implant 10 may include an implantation or shuttle suture. In some embodiments, the implantation suture may be the same or similar to the suture 12. The implantation suture may extend through or otherwise be coupled to the anchor tube 14 and may be utilized to implant the anchor tube.

As shown in FIGS. 5A-5C, the implant 10 may be implanted such that the anchor tube 14 is positioned in or through a first anatomical structure 62A. In some embodiments, the first anatomical structure 62A may be a first bone and/or tissue portion or segment. For example, as shown in FIGS. 5A-5C, the anchor tube 14 may be passed through an aperture 66 in a bone 62A such that the anchor tube 14 is positioned adjacent to or on an inner cortex surface 64 of the bone 62A (or alternatively an outer surface of the bone 62A). In such a configuration, the first and second end segments 22, 32 and the loop portion 29 (i.e., the first and second intermediate portions 26, 36) of the suture 12 may initially extend through the aperture 66 in the first anatomical structure 62A, as shown in FIG. 5A.

As shown in FIGS. 5A-5C, the first and second end segments 22, 32 of the first and second end portions 20, 32 may each be passed through a second anatomical structure 62B at least once (i.e., once or a plurality of times). In some embodiments, the second anatomical structure 62B may be a second bone and/or tissue portion or segment. For example, as shown in FIGS. 5A-5C, the second anatomical structure 62B may comprise a tissue, such as a tendon. In some such embodiments, the second anatomical structure 62B may comprise a plantar plate, and the first anatomical structure 62A may comprise a metatarsal bone. As explain above, the first and second end segments 22, 32 of the first and second end portions 20, 32 may be coupled to an insertion device(s) (e.g., one or more needles), and the insertion device(s) may be utilized to translate the first and second end segments 22, 32 through the second anatomical structure 62B.

After extending through the second anatomical structure 62B, the first and second end segments 22, 32 of the first and second end portions 20, 32 of the suture 12 may be passed through the exposed loop portions of the first and second suture passers 50A, 50B, respectively, (see FIG. 5A) and the first and second suture passers 50A, 50B translated out through the loop portion 29 such that the first and second end segments 22, 32 extend through the respective portion(s) of the loop portion 29, as shown in FIGS. 5B and 5C. As shown in FIG. 5B, the first and second end portions 20, 32 of the suture 12 may then be tensioned to slide the suture 12 through the anchor tube 14 and the first and second anatomical structures 62A, 62B such that the loop portion 29 is retracted/shortened, the anchor tube 14 is reconfigured/changed into the deformed state/configuration, and the first and second anatomical structures 62A, 62B are approximated or drawn toward each other/together (i.e., the system/construct 10 is tensioned and approximated). As explained above, the suture 12 of the loop portion 29 may act to securely grip or hold the first and second end segments 22, 32 such that the suture 12 is prevented from sliding back through the loop portion 20, and thereby back through the anchor tube 14 and the first and second anatomical structures 62A, 62B to allow the first and second anatomical structures 62A, 62B translate away from each other.

As shown in FIG. 5B, in some embodiments, the aperture 66 in the first anatomical structure 62A and/or the configuration of the first and second end segments 22, 32 passing through the portion(s) of the loop portion 29 may be such that the end of the loop portion 29 with the first and second end segments 22, 32 passing therethrough resides/becomes seated against the outer surface of the first anatomical structure 62A when the system/construct 10 is tensioned and approximated. However, in some other embodiments, as shown in FIG. 5C, the aperture 66 in the first anatomical structure 62A and/or the configuration of the first and second end segments 22, 32 passing through the portion(s) of the loop portion 29 may be such that the end of the loop portion 29 with the first and second end segments 22, 32 passing therethrough resides/becomes seated against the outer surface of the anchor tube 14 when the system/construct 10 is tensioned and approximated.

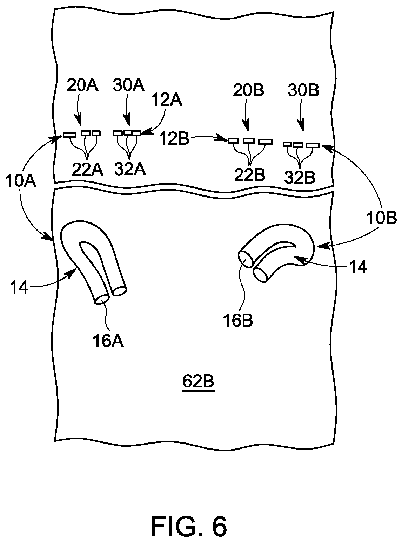

As show in FIG. 6, in some embodiments a plurality of systems/constructs 10A, 10B may be utilized to secure (and potentially draw together) first and second anatomical structures 62A, 62B together. For example, as shown in FIG. 6, a plurality of systems/constructs 10A, 10B (or a single systems/construct) may be utilized to secure (and potentially draw together) first and second tissue (e.g., ligament) segments 62A, 62B together, such as two plantar plate segments.

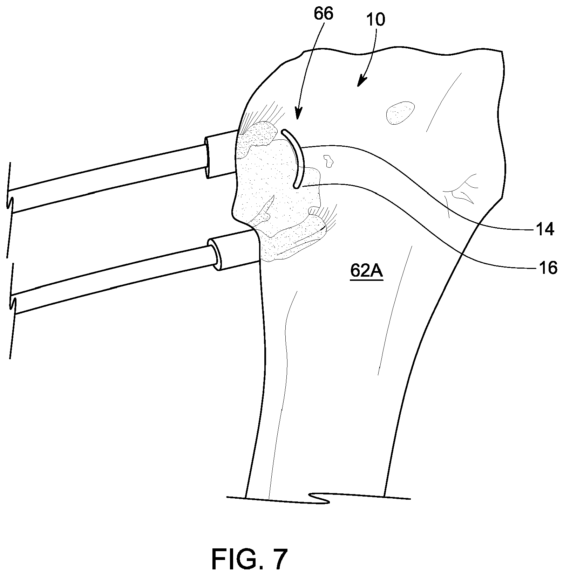

As show in FIG. 7, in some embodiments one or more system/construct 10 may be utilized to secure (and potentially draw together) a first anatomical structure 62A and a second anatomical structure (not shown) with the anchor tube 14 positioned on an exterior surface of the first anatomical structure 62A and the first and second end segments 22, 32 of the first and second end portions 20, 32 of the suture 12 extending through the first anatomical structure 62A. For example, as shown in FIG. 7, a system/construct 10 may be utilized to secure (and potentially draw together) a bone/bone segment 62A with another bone or a tissue/tissue segment with the anchor tube 14 positioned on an exterior surface of the bone 62A and the first and second end segments 22, 32 of the suture 12 extending through the bone 62A.

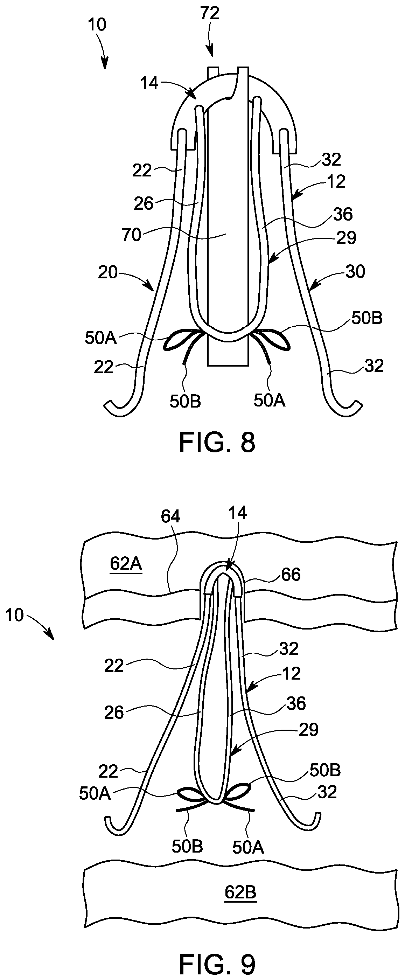

An exemplary method of utilizing the system/construct 10 to couple a tissue 62B (e.g., a plantar plate) (which may be a tissue segment) to a bone 62A (e.g., a metatarsal or phalanx bone) (which may be a bone segment) is shown in FIGS. 8-12.

As shown in FIG. 8, the anchor tube 14 may be loaded on inserter tip 72 of an inserter instrument 70. The inserter instrument 70 may be configured to hold or retain the anchor tube 14 on the inserter tip 72, such as via tension of the suture 12. As shown in FIG. 8, the inserter tip 72 may include a forked free end and a pair of grooves extending proximally from the base of the forked end along differing (e.g., opposing) sides. The forked free end and at least one groove are configured to retain or mate with the anchor tube 14 with a medial portion of the anchor tube 14 extending over the base of the forked free end between the tines or arms thereof and end portions of the anchor extending within and along the grooves, as shown in FIG. 8. The inserter 70 may be relatively stiff, and thereby utilized to insert or pass the anchor tube 14 retained on the inserter tip 72 into and through a hole 66 in the bone 62A, as shown in FIG. 9.

As also shown in FIG. 9, the aperture in the bone 62A may only extend partially through the bone 62A, and the anchor tube 14 may be positioned on/adjacent an inner surface 64 of the bone 62A, such as the inner surface of the cortex of the bone 62A. In other embodiments, the anchor tube 14 may not be positioned within the bone 62A, but may be passed through the bone 62A and positioned adjacent an exterior surface of the bone 62A (see, FIG. 7). In some embodiments, the anchor tube 14 may be positioned within the bone 62A (i.e., within the aperture 66) but not positioned on/adjacent the inner surface 64 of the bone 62A.

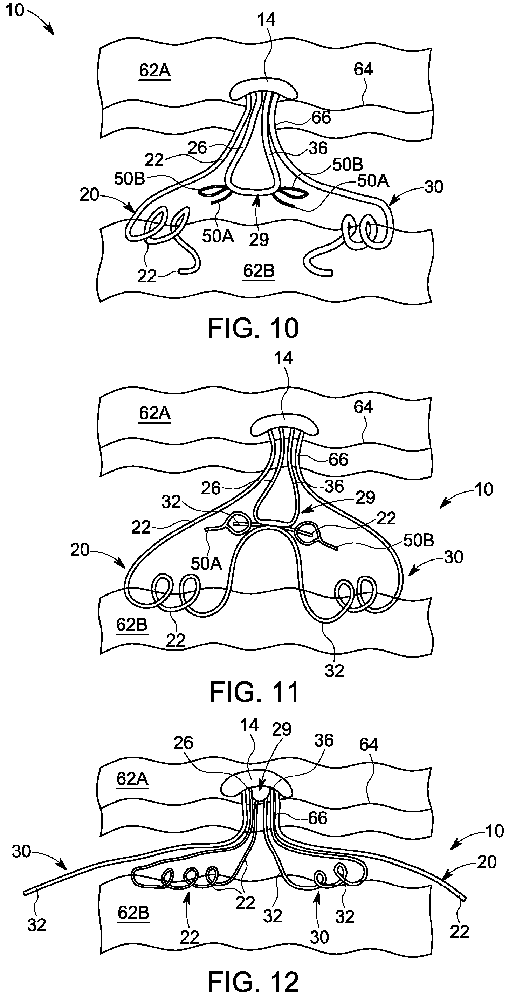

With the anchor tube 14 positioned on/in the bone 62A, the loop portion 29 and the first and second end segments 22, 32 of the first and second end portions 20, 32 of the suture 12 may extend through the aperture 66 and be exposed, as shown in FIG. 9. As shown in FIG. 10, the first and second end segments 22, 32 of the first and second end portions 20, 32 may then be passed through the tissue 62B at least once. As shown FIG. 8, the first and second end segments 22, 32 may be passed through differing portions of the tissue 62B, and/or may passed through the tissue 62B a plurality of times. As discussed above, the first and second end segments 22, 32 may be coupled to insertion needles, which may be utilized to pass the first and second end segments 22, 32 through the tissue 62B (not shown).

After the first and second end segments 22, 32 of the first and second end portions 20, 32 are passed through the tissue 62B, the first and second end segments 22, 32 may be passed through the loop or hook portions of the first and second suture passers 50A, 50B, respectively, and the first and second suture passers 50A, 50B pulled or otherwise passed through the respective portion(s) of the loop portion 29 to pass/translate the first and second end segments 22, 32 through the suture 12 of the loop portion 29, as shown in FIG. 11. It is noted that the first and second end segments 22, 32 may be disconnected from the insertion devices prior to being passed/translated through the suture 12 of the loop portion 29.

As shown in FIG. 12, after the first and second end segments 22, 32 are passed/translated through the portion(s) of the suture 12 of the loop portion 29, the first and second end portions 20, 32 of the suture 12 (e.g., the portions of the first and second end segments 22, 32 extending past the loop portion 29) may be tensioned (e.g., tensioned or drawn away from each other along divergent (e.g., opposing) directions) to slide the suture 12 through the anchor tube 14 and the tissue 62B (and potentially the bone 62A) such that the loop portion 29 is retracted/shortened, the anchor tube 14 is deformed into the deformed state/configuration, and the bone 62A and the tissue 62B are approximated/drawn toward each other/together (i.e., the system/construct 10 is tensioned and approximated) , as shown in FIG. 12. As explained above, the suture 12 of the loop portion 29 may act to securely grip or hold the first and second end segments 22, 32 such that the suture 12 is prevented from sliding back through the loop portion 20, and thereby back through the anchor tube 14 and the tissue 62B (and potentially the bone 62A) to allow the bone 62A and the tissue 62B translate away from each other. In this way, the first and second end segments 22, 32 of the suture 12 do not need to be knotted or otherwise secured together to secure the arrangement/configuration of the system/construct 10, and thereby the approximated configuration of the bone 62A and the tissue 62B, as shown in FIG. 12.

As noted above, tensioning the first and second end portions 20, 32 of the suture 12 pulls/deforms the anchor tube 14 into a concave/convex or folded arrangement or shape (as described above), as shown in FIGS. 5A-7 and 12. The deformed concave/convex or folded arrangement or shape of the anchor tube 14 assists in preventing the anchor tube 14 from passing through the aperture 66 in the bone 62A. The pulling or tensioning of the end portions/strands 22 of the tensioning suture 12 in divergent directions also firmly seats the anchor tube 14 against the surface 64 of the bone 62A, and draws the bone 62A and the tissue 62B toward each other (and potentially together). The loop portion 29 then automatically or naturally locks the position of the first and second end portions 20, 32 of the suture 12 within the loop portion 29 to lock or fix the system/construct 10, and thereby fix or secure the positional relationship of the bone 62A and the tissue 62B, as shown in FIG. 12.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has", and "having"), "include" (and any form of include, such as "includes" and "including"), and "contain" (and any form of contain, such as "contains" and "containing") are open-ended linking verbs. As a result, a method or device that "comprises," "has," "includes," or "contains" one or more steps or elements possesses those one or more steps or elements, but is not limited to possessing only those one or more steps or elements. Likewise, a step of a method or an element of a device that "comprises," "has," "includes," or "contains" one or more features possesses those one or more features, but is not limited to possessing only those one or more features. Furthermore, a device or structure that is configured in a certain way is configured in at least that way, but may also be configured in ways that are not listed.

The invention has been described with reference to the preferred embodiments. It will be understood that the architectural and operational embodiments described herein are exemplary of a plurality of possible arrangements to provide the same general features, characteristics, and general system operation. Modifications and alterations will occur to others upon a reading and understanding of the preceding detailed description. It is intended that the invention be construed as including all such modifications and alterations falling within the scope of the claims.

## Claims

1. A bone and/or tissue joining implant system (10), comprising:
a flexible anchor tube (14) comprising an annular side wall (16) defining an internal cavity (18), a first longitudinal end (17A) and a second longitudinal end (17B);
a suture (12) that extends through the side wall (16) of the anchor tube (14) a plurality of times and forms a free loop portion (29) that extends from the side wall (16); and
at least one suture passer (50A, 50B) extending through the suture (12) in the loop portion (29) that is configured to translate first and second end portions (20, 30) of the suture (12) through the loop portion (29) via translation of the at least one suture passer (50A, 50B),
wherein the suture (12) extends along a pattern that allows the suture (12) to slide through the anchor tube (14) when the first and second end portions (20, 30) of the suture are tensioned, **characterised in that** the system is configured such that tensioning of the first and second end portions (20, 30) of the suture (12) deforms the anchor tube (14) from a neutral configuration into a deformed configuration wherein the anchor tube (14) is folded over upon itself along a longitudinal length thereof.

2. The implant system (10) according to claim 1, wherein the first end portion (20) of the suture (12) extends from a first end (22) of the suture (12) and through a first portion of the side wall (16) of the anchor tube (14) from an exterior surface of the anchor tube (14) proximate to the first longitudinal end (17A) of the anchor tube (14) into the internal cavity (18), extends longitudinally through a portion of the internal cavity (18) toward the second longitudinal end (17B) of the anchor tube (14), and extends through a second portion of the side wall (16) from the internal cavity (18) to the exterior surface of the anchor tube (14), the second portion of the side wall being longitudinally spaced further from the first longitudinal end (17A) than the first portion of the side wall (16).

3. The implant system (10) according to claim 2, wherein the second end portion of the suture (12) extends from a second end (32) of the suture (12) and through a third portion of the side wall (16) from an exterior surface of the anchor tube (14) proximate to the second longitudinal end (17B) of the anchor tube (14) into the internal cavity (18), extends longitudinally through a portion of the internal cavity (18) toward the first longitudinal end (17A), and extends through a fourth portion of the side wall (16) from the internal cavity (18) to the exterior surface of the anchor tube (14), the fourth portion of the side wall (16) being longitudinally spaced further from the second longitudinal end (17B) than the third portion of the side wall (16).

4. The implant system (10) according to claim 3, wherein a first intermediate portion (26) of the suture (12) extends from the first end portion (20) that extends through the second portion of the side wall (16) of the anchor tube (14), and a second intermediate portion (36) of the suture (12) extends from the second end portion (32) that extends through the fourth portion of the side wall (16) of the anchor tube (14), and wherein the first and second intermediate portions (26, 36) of the suture (12) form the loop portion (29).

5. The implant system (10) according to claim 4, wherein the first and second intermediate portions (26, 36) of the suture (12) are contiguous portions of the suture (12).

6. The implant system (10) according to claim 1, wherein, in the deformed configuration, the anchor tube (14) defines a curved shape.

7. The implant system (10) according to claim 6, wherein the curved shape comprises a U-shape or a V-shape.

8. The implant system (10) according to claim 1, wherein the anchor tube (14) defines a first maximum lateral width and a first maximum longitudinal length in the neutral configuration, and defines a second maximum lateral width that is greater than the first maximum lateral width and a second maximum longitudinal length that is less than the first maximum longitudinal length in the deformed configuration.

9. The implant system (10) according to any of the preceding claims, wherein the anchor tube (14) is longitudinally elongated and cylindrical in the neutral configuration.

10. The implant system (10) according to any of the preceding claims, wherein the anchor tube (14) and the suture (12) are formed from a biocompatible material.

11. The implant system according to any of the preceding claims, wherein the suture (12) is a multifilament suture (12).

12. The implant system (10) according to claim 11, wherein the suture (12) is a braided suture (12).

13. The implant system (10) according to any of the preceding claims, wherein the suture (12) is non-hollow.

14. The implant system (10) according to any of the preceding claims, wherein the anchor tube (14) is flexible and formed of a hollow braided suture.

15. The implant system (10) according to any of the preceding claims, wherein, with the first and second end portions (20, 30) translated through the loop portion (29) via the at least one suture passer (50A, 50B), the tensioning of the first and second end portions (20, 30) of the suture (12) shortens the loop portion (29) such that the loop portion (29) creates sufficient friction and/or compression on the first and second end portions (20, 30) to prevent them from sliding back through the loop portion (29).

## Patentansprüche

1. Ein Knochen und/oder Gewebe verbindendes Implantatsystem (10), beinhaltend:
eine flexible Verankerungsröhre (14), beinhaltend eine ringförmige Seitenwand (16), die einen inneren Hohlraum (18), ein erstes Longitudinalende (17A) und ein zweites Longitudinalende (17B) definiert;
einen Faden (12), der sich mehrfach durch die Seitenwand (16) der Verankerungsröhre (14) erstreckt und einen freien Schleifenabschnitt (29) bildet, der sich von der Seitenwand (16) erstreckt; und
mindestens einen Fadendurchgänger (50A, 50B), der sich in dem Schleifenabschnitt (29) durch den Faden (12) erstreckt und konfiguriert ist, um einen ersten und einen zweiten Endabschnitt (20, 30) des Fadens (12) mittels Translation des mindestens einen Fadendurchgängers (50A, 50B) durch den Schleifenabschnitt (29) zu translatieren,
wobei sich der Faden (12) entlang eines Musters erstreckt, das es dem Faden (12) ermöglicht, durch die Verankerungsröhre (14) zu gleiten, wenn der erste und der zweite Endabschnitt (20, 30) des Fadens gespannt werden, **dadurch gekennzeichnet, dass** das System so konfiguriert ist, dass
Spannen des ersten und des zweiten Endabschnitts (20, 30) des Fadens (12) die Verankerungsröhre (14) aus einer neutralen Konfiguration in eine verformte Konfiguration verformt, wobei die Verankerungsröhre (14) entlang einer longitudinalen Länge auf sich selbst zurückgefaltet wird.

2. Implantatsystem (10) gemäß Anspruch 1, wobei sich der erste Endabschnitt (20) des Fadens (12) von einem ersten Ende (22) des Fadens (12) und von einer äußeren Oberfläche der Verankerungsröhre (14) nahe dem ersten Longitudinalende (17A) der Verankerungsröhre (14) durch einen ersten Abschnitt der Seitenwand (16) der Verankerungsröhre (14) in den inneren Hohlraum (18) erstreckt, sich longitudinal durch einen Abschnitt des inneren Hohlraums (18) in Richtung des zweiten Longitudinalendes (17B) der Verankerungsröhre (14) erstreckt und sich von dem inneren Hohlraum (18) durch einen zweiten Abschnitt der Seitenwand (16) zu der äußeren Oberfläche der Verankerungsröhre (14) erstreckt, wobei der zweite Abschnitt der Seitenwand weiter von dem ersten Longitudinalende (17A) longitudinal beabstandet ist als der erste Abschnitt der Seitenwand (16).

3. Implantatsystem (10) gemäß Anspruch 2, wobei sich der zweite Endabschnitt des Fadens (12) von einem zweiten Ende (32) des Fadens (12) und von einer äußeren Oberfläche der Verankerungsröhre (14) nahe dem zweiten Longitudinalende (17B) der Verankerungsröhre (14) durch einen dritten Abschnitt der Seitenwand (16) in den inneren Hohlraum (18) erstreckt, sich longitudinal durch einen Abschnitt des inneren Hohlraums (18) in Richtung des ersten Longitudinalendes (17A) erstreckt und sich von dem inneren Hohlraum (18) durch einen vierten Abschnitt der Seitenwand (16) zu der äußeren Oberfläche der Verankerungsröhre (14) erstreckt, wobei der vierte Abschnitt der Seitenwand (16) weiter von dem zweiten Longitudinalende (17B) longitudinal beabstandet ist als der dritte Abschnitt der Seitenwand (16).

4. Implantatsystem (10) gemäß Anspruch 3, wobei sich ein erster Zwischenabschnitt (26) des Fadens (12) von dem ersten Endabschnitt (20), der sich durch den zweiten Abschnitt der Seitenwand (16) der Verankerungsröhre (14) erstreckt, erstreckt und sich ein zweiter Zwischenabschnitt (36) des Fadens (12) von dem zweiten Endabschnitt (32), der sich durch den vierten Abschnitt der Seitenwand (16) der Verankerungsröhre (14) erstreckt, erstreckt und wobei der erste und der zweite Zwischenabschnitt (26, 36) des Fadens (12) den Schleifenabschnitt (29) bilden.

5. Implantatsystem (10) gemäß Anspruch 4, wobei der erste und der zweite Zwischenabschnitt (26, 36) des Fadens (12) zusammenhängende Abschnitte des Fadens (12) sind.

6. Implantatsystem (10) gemäß Anspruch 1, wobei die Verankerungsröhre (14) in der verformten Konfiguration eine gekrümmte Form definiert.

7. Implantatsystem (10) gemäß Anspruch 6, wobei die gekrümmte Form eine U-Form oder eine V-Form beinhaltet.

8. Implantatsystem (10) gemäß Anspruch 1, wobei die Verankerungsröhre (14) in der neutralen Konfiguration eine erste maximale laterale Breite und eine erste maximale longitudinale Länge definiert und in der verformten Konfiguration eine zweite maximale laterale Breite, die größer als die erste maximale laterale Breite ist, und eine zweite maximale longitudinale Länge, die kleiner als die erste maximale longitudinale Länge ist, definiert.

9. Implantatsystem (10) gemäß einem der vorhergehenden Ansprüche, wobei die Verankerungsröhre (14) in der neutralen Konfiguration longitudinal verlängert und zylindrisch ist.

10. Implantatsystem (10) gemäß einem der vorhergehenden Ansprüche, wobei die Verankerungsröhre (14) und der Faden (12) aus einem biokompatiblen Material gebildet sind.

11. Implantatsystem gemäß einem der vorhergehenden Ansprüche, wobei der Faden (12) ein Multifilamentfaden (12) ist.

12. Implantatsystem (10) gemäß Anspruch 11, wobei der Faden (12) ein geflochtener Faden (12) ist.

13. Implantatsystem (10) gemäß einem der vorhergehenden Ansprüche, wobei der Faden (12) nicht hohl ist.

14. Implantatsystem (10) gemäß einem der vorhergehenden Ansprüche, wobei die Verankerungsröhre (14) flexibel und aus einem hohlen geflochtenen Faden gebildet ist.

15. Implantatsystem (10) gemäß einem der vorhergehenden Ansprüche, wobei, wenn der erste und der zweite Endabschnitt (20, 30) mittels des mindestens einen Fadendurchgängers (50A, 50B) durch den Schleifenabschnitt (29) translatiert werden, das Spannen des ersten und des zweiten Endabschnitts (20, 30) des Fadens (12) den Schleifenabschnitt (29) verkürzt, sodass der Schleifenabschnitt (29) ausreichend Reibung und/oder Druck auf den ersten und den zweiten Endabschnitt (20, 30) erzeugt, um sie davon abzuhalten, durch den Schleifenabschnitt (29) zurück zu gleiten.

## Revendications

1. Un système d'implant de liaison d'os et/ou de tissu (10), comprenant :
un tube d'ancrage (14) souple comprenant une paroi latérale annulaire (16) définissant une cavité interne (18), une première extrémité longitudinale (17A) et une deuxième extrémité longitudinale (17B) ;
un fil de suture (12) qui s'étend à travers la paroi latérale (16) du tube d'ancrage (14) une pluralité de fois et forme une portion formant boucle (29) libre qui s'étend depuis la paroi latérale (16) ; et
au moins un passe-suture (50A, 50B) s'étendant à travers le fil de suture (12) dans la portion formant boucle (29) qui est configuré pour translater des première et deuxième portions d'extrémité (20, 30) du fil de suture (12) à travers la portion formant boucle (29) par l'intermédiaire d'une translation de l'au moins un passe-suture (50A, 50B),
dans lequel le fil de suture (12) s'étend le long d'un motif qui permet au fil de suture (12) de glisser à travers le tube d'ancrage (14) lorsque les première et deuxième portions d'extrémité (20, 30) du fil de suture sont mises en tension, **caractérisé en ce que** le système est configuré de telle sorte que
la mise en tension des première et deuxième portions d'extrémité (20, 30) du fil de suture (12) déforme le tube d'ancrage (14) d'une configuration neutre en une configuration déformée dans laquelle le tube d'ancrage (14) est replié sur lui-même sur une longueur longitudinale de celui-ci.

2. Le système d'implant (10) selon la revendication 1, dans lequel la première portion d'extrémité (20) du fil de suture (12) s'étend d'une première extrémité (22) du fil de suture (12) et à travers une première portion de la paroi latérale (16) du tube d'ancrage (14) depuis une surface extérieure du tube d'ancrage (14) à proximité de la première extrémité longitudinale (17A) du tube d'ancrage (14) jusque dans la cavité interne (18), s'étend longitudinalement à travers une portion de la cavité interne (18) vers la deuxième extrémité longitudinale (17B) du tube d'ancrage (14), et s'étend à travers une deuxième portion de la paroi latérale (16) depuis la cavité interne (18) jusqu'à la surface extérieure du tube d'ancrage (14), la deuxième portion de la paroi latérale étant longitudinalement davantage espacée de la première extrémité longitudinale (17A) que la première portion de la paroi latérale (16).

3. Le système d'implant (10) selon la revendication 2, dans lequel la deuxième portion d'extrémité du fil de suture (12) s'étend d'une deuxième extrémité (32) du fil de suture (12) et à travers une troisième portion de la paroi latérale (16) depuis une surface extérieure du tube d'ancrage (14) à proximité de la deuxième extrémité longitudinale (17B) du tube d'ancrage (14) jusque dans la cavité interne (18), s'étend longitudinalement à travers une portion de la cavité interne (18) vers la première extrémité longitudinale (17A), et s'étend à travers une quatrième portion de la paroi latérale (16) depuis la cavité interne (18) jusqu'à la surface extérieure du tube d'ancrage (14), la quatrième portion de la paroi latérale (16) étant longitudinalement davantage espacée de la deuxième extrémité longitudinale (17B) que la troisième portion de la paroi latérale (16).

4. Le système d'implant (10) selon la revendication 3, dans lequel une première portion intermédiaire (26) du fil de suture (12) s'étend depuis la première portion d'extrémité (20) qui s'étend à travers la deuxième portion de la paroi latérale (16) du tube d'ancrage (14), et une deuxième portion intermédiaire (36) du fil de suture (12) s'étend depuis la deuxième portion d'extrémité (32) qui s'étend à travers la quatrième portion de la paroi latérale (16) du tube d'ancrage (14), et les première et deuxième portions intermédiaires (26, 36) du fil de suture (12) formant la portion formant boucle (29).

5. Le système d'implant (10) selon la revendication 4, dans lequel les première et deuxième portions intermédiaires (26, 36) du fil de suture (12) sont des portions contiguës du fil de suture (12).

6. Le système d'implant (10) selon la revendication 1, dans lequel, dans la configuration déformée, le tube d'ancrage (14) définit une forme courbe.

7. Le système d'implant (10) selon la revendication 6, dans lequel la forme courbe comprend une forme en U ou une forme en V.

8. Le système d'implant (10) selon la revendication 1, dans lequel le tube d'ancrage (14) définit une première largeur latérale maximale et une première longueur longitudinale maximale dans la configuration neutre, et définit une deuxième largeur latérale maximale qui est supérieure à la première largeur latérale maximale et une deuxième longueur longitudinale maximale qui est inférieure à la première longueur longitudinale maximale dans la configuration déformée.

9. Le système d'implant (10) selon n'importe lesquelles des revendications précédentes, dans lequel le tube d'ancrage (14) est longitudinalement allongé et cylindrique dans la configuration neutre.

10. Le système d'implant (10) selon n'importe lesquelles des revendications précédentes, dans lequel le tube d'ancrage (14) et le fil de suture (12) sont formés à partir d'un matériau biocompatible.

11. Le système d'implant selon n'importe lesquelles des revendications précédentes, dans lequel le fil de suture (12) est un fil de suture multifilament (12).

12. Le système d'implant (10) selon la revendication 11, dans lequel le fil de suture (12) est un fil de suture tressé (12).

13. Le système d'implant (10) selon n'importe lesquelles des revendications précédentes, dans lequel le fil de suture (12) n'est pas creux.

14. Le système d'implant (10) selon n'importe lesquelles des revendications précédentes, dans lequel le tube d'ancrage (14) est souple et formé d'un fil de suture tressé creux.

15. Le système d'implant (10) selon n'importe lesquelles des revendications précédentes, dans lequel, avec les première et deuxième portions d'extrémité (20, 30) translatées à travers la portion formant boucle (29) par l'intermédiaire de l'au moins un passe-suture (50A, 50B), la mise en tension des première et deuxième portions d'extrémité (20, 30) du fil de suture (12) raccourcit la portion formant boucle (29) de telle sorte que la portion formant boucle (29) crée un frottement et/ou une compression suffisants sur les première et deuxième portions d'extrémité (20, 30) pour les empêcher de glisser de nouveau à travers la portion formant boucle (29).
